# EUROPEAN PATENT SPECIFICATION

(11) **EP 2 471 475 B1**
(45) Date of publication and mention of the grant of the patent: **17.10.2018**
(21) Application number: 10811866.2
(22) Date of filing: 24.08.2010
(51) Int. Cl.: A61B 17/34, A61F 9/007, A61M 5/158, A61M 25/00

(54) **CANNULA AND METHOD FOR PRODUCING SAME**
KANÜLE UND HERSTELLUNGSVERFAHREN DAFÜR
CANULE ET SON PROCÉDÉ DE PRODUCTION

(30) Priority: 28.08.2009 JP 2009197630
(43) Date of publication of application: 04.07.2012
(73) Proprietor: Mani, Inc., Tochigi 321-3231 (JP)
(72) Inventor: MURAKAMI Etsuo, Utsunomiya-shi Tochigi 321-3231 (JP); OGANE Kaoru, Utsunomiya-shi Tochigi 321-3231 (JP)
(74) Representative: Westphal, Mussgnug & Partner Patentanwälte mbB
(86) International application number: PCT/JP2010/064304
(87) International publication number: WO 2011/024816

(56) References cited:
- EP-A1- 1 923 010
- JP-A- 3 251 357
- JP-A- 7 016 235
- JP-A- H0 716 235
- US-A- 5 300 036
- US-A- 5 300 036
- US-A- 5 697 913
- US-A1- 2004 106 942
- US-A1- 2009 192 444

## Description

### TECHNICAL FIELD

The present invention relates to a cannula, which constitutes a trocar inserted through tissue in surgery and forming a passage through which surgical instruments and tubes for injecting chemicals reach an affected area. Also described, but not claimed is a method of manufacturing the cannula.

### BACKGROUND ART

A trocar is adapted to have a tubular cannula. A portion of body surface tissue is cut out to make the cannula penetrate and, thus, to arrange the cannula, so that the trocar forms a passage for passing surgical instruments and light fixture required for surgery for the affected area or tubes for injecting chemicals.

In an ophthalmic vitreous surgery, for example, there are used a plurality of trocars including a trocar for inserting a tube for injecting saline for keeping eye pressure, a trocar for inserting surgical instruments typified by forceps and scissors for incising and removing the affected area, and a trocar for inserting a probe for irradiating laser beam to the affected area. Those trocars are penetrated through a hole formed by incising a conjunctiva and a sclera to insert an end of a cannula into a vitreous body, necessary instruments and tubes are inserted in this state, and intended surgery is performed.

As described above, although it is essential to incise a conjunctiva and a sclera in vitreous surgery, the diameter of a cannula is extremely small, and therefore, a cutting instrument is previously inserted through inside the cannula, a conjunctiva and a sclera are incised by the cutting instrument, and then the cannula is inserted into a vitreous body. After the cannula is inserted into the vitreous body, only the cutting instrument is drawn out to leave the cannula.

In eye surgery, in order to reduce a period required for curing, an incised portion can be closed without suturing. Thus, the invention described in Patent Document 1 is proposed. In this technique, a conjunctiva and/or a sclera can be saved so that an opening (incisional wound) formed within the eye for the execution of intraocular surgical procedures can be closed by the tissue of the opening after the surgical procedures. With the technique, a small opening is realized by using a surgical instrument whose operable end has such an extremely small diameter as 20 to 27 gauges, so that the tissue of the opening can be closed.

In the above trocars, the end of the cannula through which the cutting instrument is inserted is formed in a state of being cut at right angles with respect to an axis line. Namely, the thickness of the cannula as a flat surface is exposed at the end of the cannula. Thus, the end of the cannula is screwed into and penetrates through a conjunctiva and a sclera incised by the cutting instrument. Accordingly, the tissues around the incisional wound may be dissected. If the tissues are dissected, a problem that it takes time to cure completely may occur.

In order to solve the problems, the invention described in Patent Document 2 is proposed. In this technique, an oblique cut surface is formed at the end of the cannula constituting the trocar, and the oblique cut surface is formed into a tapered shape. In the technique, the oblique cut surface formed at the end of the cannula has an inclined surface corresponding to the taper from the inner peripheral surface side of the cannula toward the outer peripheral surface side.

Thus, in order to have the cannula penetrate through an incisional wound formed in tissue by a cutting instrument mounted to the cannula, the cutting instrument and the tapered portion formed at a pointed portion of the oblique cut surface are first passed through the incisional wound. After that, a thick portion of the taper is passed through the incisional wound while following the inclination of the oblique cut surface. Namely, since the cannula penetrates through the incisional wound so that the incisional wound is pushed and opened by the oblique cut surface and the taper, there is no possibility that the tissue is dissected.

### PRIOR ART DOCUMENTS

### PATENT DOCUMENTS

Patent Document 1: Japanese Patent Application National Publication (Laid-Open) No. 2003-526461
Patent Document 2: Japanese Patent Application Laid-Open No. 2008-142533

Cylindrical cannulas which are a part of the trocar used in surgery and into which a cutting member is inserted in detachable manner are known for example from EP 1 923 010 A1, JP H 07 16235 A, US 5,697,913 A, and US 2004/106942 A1.

US 5, 300, 036 A may disclose a cannula with the features of the preamble of claim 1.

### DISCLOSURE OF THE INVENTION

### PROBLEMS TO BE SOLVED BY THE INVENTION

In the cannula constituting the trocar described in the Patent Document 2, when the cannula penetrates through an incisional wound formed in tissue, there is no possibility that the tissue is dissected. However, even this technique is not complete yet, and there are some problems to be improved.

For example, since the taper formed on the oblique cut surface formed at the end of the cannula is formed on the basis of the central axis of the oblique cut surface, there is increased the angle of the tapered surface at the end of the cannula relative to an axial center of a cylindrical body constituting the cannula. Therefore, there is increased the rate of the increase in diameter with respect to the length that the oblique cut surface passes through the incisional wound when the cannula is penetrated through the incisional wound. Accordingly, the rate of the increase in resistance generated in impaling (impalement resistance) is increased.

In eye surgery requiring a delicate operation, an incisional wound is formed in a conjunctiva and a sclera by a cutting member, and when a cannula is then penetrated through the incisional wound, it is not preferable that the power required for the operation significantly varies. Namely, if the power required for the operation is largely varied, the cannula may be penetrated to an excessive depth.

An object of the present invention is to provide a cannula which can reduce the rate of the increase in resistance generated when the cannula is penetrated through an incisional wound formed in tissue.

### SOLUTIONS TO THE PROBLEMS

A cannula according to the present invention for solving the above problem is a cylindrical cannula constituting a trocar used in a surgery and inserting thereinto a cutting member in a detachably attachable manner. In the cannula, an inclined cut surface in which one side of a cylinder protrudes is formed at the end of the cannula. A tapered surface with uneven width, where the protruding side of the inclined cut surface has a larger width than the nonprotruding side, is formed on an outer peripheral surface of the inclined cut surface.

Further, a cannula manufacturing method for manufacturing a cannula according to the present invention is described, but not claimed. In the cannula manufacturing method, an inclined cut surface in which one side of a cylinder protrudes is formed at an end of a cylindrical material. When the outer peripheral surface of the inclined cut surface is ground or polished, the angle of the axial center of the cylinder relative to a ground surface or a polished surface is changed corresponding to the progression of grinding or polishing. Therefore, a tapered surface with uneven width, where the protruding side of the
inclined cut surface has a larger width than the nonprotruding side, is formed on the outer peripheral surface of the inclined cut surface.

### EFFECTS OF THE INVENTION

In the cannula according to the present invention, an inclined cut surface in which one side of a cylindrical material more protrudes along the cylinder than the other side is formed at an end of the cylindrical material. A tapered surface with uneven width, where the protruding side of the cylindrical material has a larger width than the nonprotruding side, is formed on the outer peripheral surface of the inclined cut surface. Namely, since the width of the tapered surface on the protruding side (the width in a direction along the axis of the cylinder) is larger than the width of the tapered surface on the nonprotruding side, the angle of the tapered surface on the protruding side relative to the axial center of the cylindrical material can be made smaller than the angle of the tapered surface on the nonprotruding side relative to the axial center of the cylindrical material.

Thus, when the cutting member is inserted through the inside of the cannula, the angle formed by the outer peripheral surface of the cutting member and the tapered surface on the protruding side of the cylindrical member becomes small. Accordingly, when the cannula passes through an incisional wound formed in tissue by the cutting member, the impalement resistance at the time when the protruding side of the inclined cut surface passes can be made small. After the protruding side of the inclined cut surface passes through the incisional wound, as the passage length of the inclined cut surface increases, from the protruding side to the nonprotruding side passes sequentially. At that time, there is reduced the rate of the increase in the outer diameter size of the inclined cut surface corresponding to the length that the inclined cut surface passes.

Accordingly, the rate of the increase in the impalement resistance accompanying the passage of the inclined cut surface can be reduced, and when the incisional wound is formed in tissue and, the operation of passing the cannula through the incisional wound is performed, the variation of the power required for the operation can be reduced.

In the cannula manufacturing method that is described, but not claimed, an inclined cut surface in which one side of a cylinder protrudes along the cylinder is formed at an end of a cylindrical material constituting a cannula. When the outer peripheral surface of the inclined cut surface is ground or polished, the angle of the axial center of the cylindrical material relative to a ground surface or a polished surface is changed corresponding to the progression of the grinding or polishing. Accordingly, a tapered surface with uneven width, where the protruding side of the inclined cut surface has a larger width than the nonprotruding side, can be formed on the outer periphery surface of the inclined cut surface.

### BRIEF DESCRIPTION OF THE DRAWINGS

FIG. 1 is a view for describing a configuration of a cannula.
FIG. 2 is a perspective view for describing a configuration of a trocar.
FIG. 3 is a view for describing a relationship between a cutting member and the cannula constituting the trocar.
FIG. 4 is an explanatory view of an enlarged relevant part of FIG. 3.
FIG. 5 is a view for describing a method of manufacturing the cannula.

### DESCRIPTION OF REFERENCE SIGNS

- A: Cannula member
- B: Trocar
- C: Cutting member
- D: Handle
- 1: Cannula
- 1a: Axial center
- 2: Inclined cut surface
- 2a, 2b: Point
- 3: Tapered portion
- 4: Cut-out line
- 10: Socket
- 20: Main body
- 20a: Tale portion
- 21: Cutting blade
- 25: Material
- 30: Grinding belt
- 31: Supply portion
- 32: Winding portion
- 33: Transfer portion

### DESCRIPTION OF PREFERRED EMBODIMENTS

Hereinafter, a configuration of a cannula according to the present invention and a method of manufacturing the cannula will be described. The cannula according to the present invention constitutes a trocar which forms an incisional wound in tissue by means of a cutting member mounted in a detachably attachable manner and impales the incisional wound to form a passage through which surgical instruments and tubes for injecting chemicals reach an affected area.

The cannula of the present invention has been made based on the following finding. Namely, when the outer diameter and the inner diameter of the cannula have the same value (the thicknesses are equal to each other), the maximum values of impalement resistance generated when the cannula passes through tissue are the same regardless of the shape of an end of the cannula. However, the rate of the increase in resistance value accompanying increase in a passage length at the time when the end of the cannula passes through tissue is changed according to the shape of the end of the cannula. When a doctor performs a surgery, the doctor can perform a more stable operation if the rate of change in the resistance value is small.

In the cannula of the present invention, when the cannula is arranged to penetrate through tissue, the cannula is required to have a strength that can keep the shape against the force from the tissue. In this case, both of the cannula having enough strength to avoid deformation of the cannula when a cutting member is pulled out and the cannula having enough strength to allow the deformation of the cannula can be used. Materials that can exhibit such strength include metal typified by stainless steel and a synthetic resin, and a pipe formed of these materials can be preferably used. Especially when a tool inserted into the cannula is moved in use, a deformable cannula is preferably used.

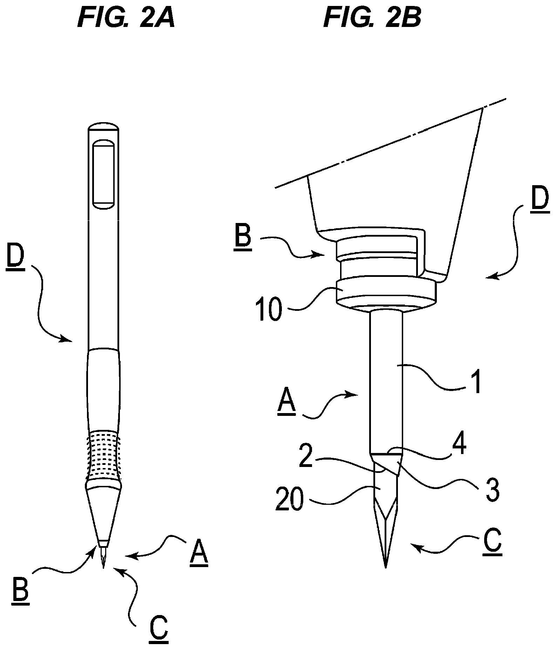

Before describing the configuration of the cannula 1 according to the present embodiment, a configuration of a trocar B provided with a cannula member A having the cannula 1 will be described using FIG. 2. The trocar B illustrated in FIG. 2 is adapted to be used in eye surgery and incises a conjunctiva and a sclera to insert the cannula member A through the incisional wound, and, thus, to leave the cannula member A. Using the cannula member A, surgical instruments and a tube can be inserted and passed through.

The trocar B is configured so that the cannula member A mounted with a cutting member C in a detachably attachable manner is further mounted to a handle D in a detachably attachable manner. A doctor grips and operates the handle D to incise a target portion of a living organism and, thus, to form an incisional wound. Then, accompanying further operation, the cannula member A is moved with the cutting member C and inserted through the incisional wound. After that, the handle D is released from the cannula member A and, in addition, the cutting member C is released from the cannula member A, therefore a hole penetrating through the incisional wound can be formed by the cannula member A.

According to the above constitution, as illustrated in FIG. 2A, the handle D has a length and thickness large enough to be gripped and operated by a doctor and is formed into a shape that is easily gripped. Further, as illustrated in FIG. 2B, an end of the handle D can be mounted by engaging with a cut-out portion and a groove of a socket 10 of the cannula member A. The handle D can be released by releasing the engagement.

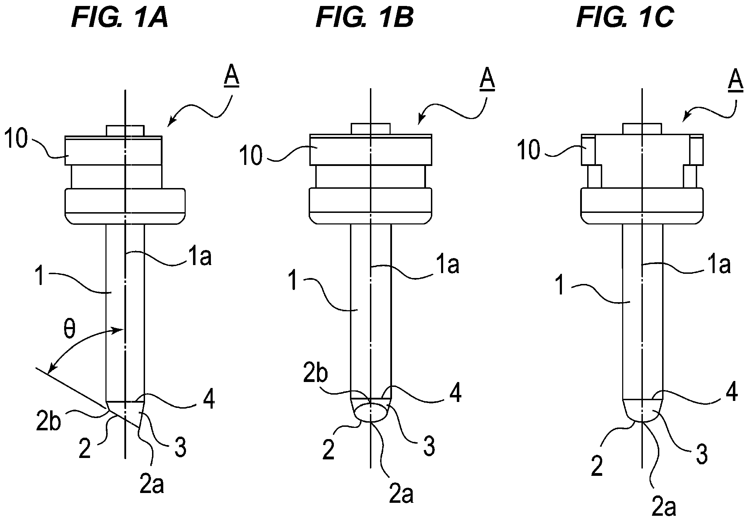

Next, the configuration of the cannula 1 according to the present embodiment will be described using FIGS. 1, 3, and 4. The cannula member A illustrated in the drawings is adapted to have the cannula 1 formed into a cylindrical shape and mounted with the cutting member C inside in a detachably attachable manner, and the socket 10 having the cannula 1 attached. The cannula 1 includes a metal pipe or a synthetic resin pipe having a rigidity, has an inclined cut surface 2 formed at the end, and is fixed to the socket 10 in such a state that the end on the other side is fitted and inserted into the socket 10.

The cannula 1 penetrates through an incisional wound in tissue formed by the cutting member C in a surgery for the affected area, and forms a passage through which surgical instruments and tubes are inserted while maintaining the penetration state. Thus, the cannula 1 includes a cylindrical member having a suitable rigidity.

The inclined cut surface 2, in which one side of a cylinder protrudes, is formed at the end of the cannula 1 and a tapered portion 3 is formed on an outer peripheral surface of the inclined cut surface 2. In the inclined cut surface 2, a circumferential point 2a at an end of the cylindrical member constituting the cannula 1 is the top, and a point 2b on the opposite side of the point 2a is the bottom. Namely, a portion corresponding to the point 2a is the protruding side, and a portion corresponding to the point 2b is the nonprotruding side.

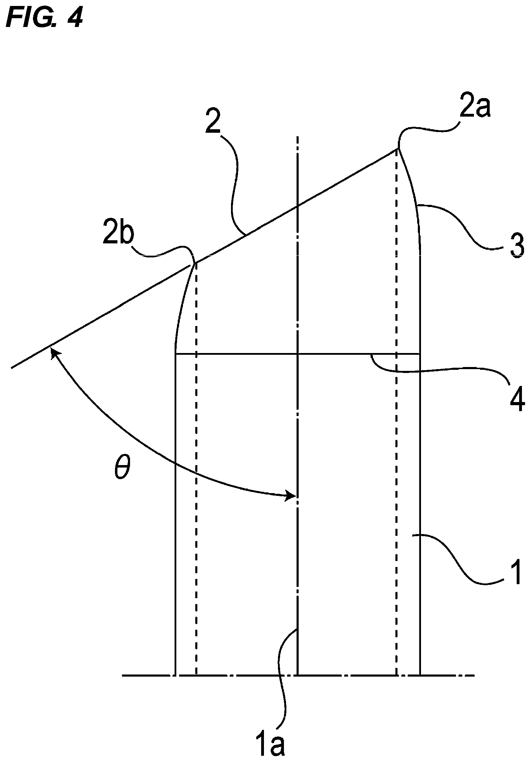

In the present invention, although an angle θ relative to an axial center 1a of the cannula 1 of the inclined cut surface 2 is not limited, the angle of about 60 degrees is formed in the present embodiment (see, FIG. 4).

In the present embodiment, although the inclined cut surface 2 is formed linearly, it is not limited to this constitution. Any shape may be used as far as the point 2a at the end of the cylindrical member constituting the cannula 1 more protrudes than the other circumferential portion. Namely, in the lateral view having the point 2a of the cannula 1 at the right side, the shape of the inclined cut surface 2 may be linear as illustrated in FIG. 4. The shape in lateral view of the inclined cut surface 2 may be curved or may be a shape in which straight lines and curved lines are combined.

The tapered portion 3 is formed on the outer peripheral surface of the inclined cut surface 2. The tapered portion 3 is formed so that the center coincides with the axial center 1a of the cannula 1 as much as possible. Further, the tapered portion 3 is formed so that a cut-out line 4, which is a border between the tapered portion 3 and the outer peripheral surface of the cannula 1, is substantially at right angle to the axial center 1a.

The tapered portion 3 is not formed to have a uniform taper angle over the entire circumference of the outer peripheral surface of the inclined cut surface 2, but formed to have different tapered angles and different tapered surfaces over the entire circumferences. Namely, the length from the point 2a to the cut-out line 4 that is the width on the protruding side constituting the inclined cut surface 2 is larger than the length from the point 2b to the cut-out line 4 that is the width on the nonprotruding side. Thus, the size from the circumference of the inclined cut surface 2 to the cut-out line 4 that is the width of the tapered portion 3 is not uniform over the entire circumference.

In the tapered portion 3, the taper angle (formed by the axial center 1a and the tapered surface) relative to the axial center 1a of the cannula 1 is not uniform but is different over the entire circumference. Namely, the angle formed by the tapered surface on the point 2a side that is the protruding side in the tapered portion 3 and the axial center 1a is smaller than the angle formed by the tapered surface on the point 2b side that is the nonprotruding side and the axial center 1a.

The shape of the tapered surface formed on the entire outer peripheral surface of the tapered portion 3 is not a straight line in the lateral view but is a curved line. Specifically, the curved line is not a simple curved line with a prescribed curvature but a complex curved line whose curvature is changed corresponding to a direction along the axial center 1a. Such a tapered surface can be formed by using a manufacturing method to be described later.

The tapered portion 3 is configured as described above, accordingly the inclination angle of the tapered portion 3 relative to the axial center 1a of the cannula 1 become small, and a reduced rate of the increase in the outer diameter with respect to the axial center 1a, from the point 2a constituting the inclined cut surface 2 to the cut-out line 4 through the point 2b, can be achieved. Thus, when the cannula 1 is penetrated through an incisional wound formed in tissue, the rate of the increase in the resistance generated when the point 2a of the inclined cut surface 2 passes through the incisional wound, the inclined cut surface 2 passes through the incisional wound sequentially from the point 2a toward the point 2b, and the cut-out line 4 further passes through the incisional wound is reduced corresponding to the rate of the increase in the outer diameter.

Accordingly, after forming the incisional wound in tissue by the cutting member C operated by a doctor, when the cannula 1 is penetrated through the incisional wound with the subsequent operation, the resistance increases as the inclined cut surface 2 starts to pass through the incisional wound. However, since the angle of the tapered portion 3 corresponding to the point 2a that is the protruding side of the inclined cut surface 2 is small, the increase in the resistance is small. Then, although the resistance increases with the passage of the tapered portion 3, the rate of increase is small, and variation of the force required for operation can be reduced. The resistance reaches the maximum value at the time of the penetration of the cannula 1 with the passage of the cut-out line 4, and the maximum value is the same regardless of the shape of the end of the cannula 1 if the inner diameter and the outer diameter of the cannula 1 are the same.

As described above, in the cannula 1 of the present embodiment, the cannula 1 is not required to be rotated when the cannula 1 is penetrated through the incisional wound formed by the cutting member C, and therefore, tissue is not dissected. Thus, a cure period after a surgery does not become longer.

The socket 10 is mounted to the handle D in a detachably attachable manner, and as described above, the cannula 1 inserting therethrough the cutting member C is mounted to the handle D through the socket 10. A doctor operates the handle D, so that an incisional wound is formed in tissue by the cutting member C, and the cannula 1 can be penetrated through the tissue with further insertion of the cutting member C into the tissue.

After that, the socket 10 is released from the handle D, and, in addition, the cutting member C is pulled out from the cannula 1, so that the cannula member A (the cannula 1) is left in the incisional wound to thereby form a passage reaching an affected area. Through the formed passage, the surgery for the affected area using surgical instruments can be performed, and chemicals can be supplied through tubes.

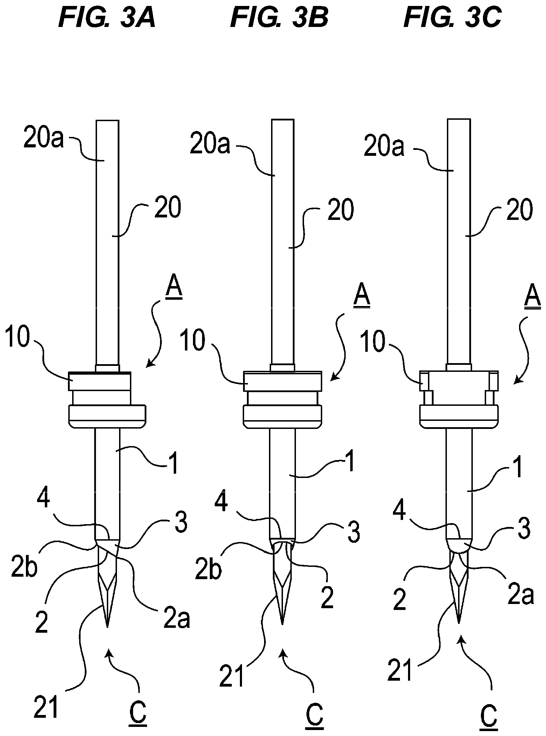

As illustrated in FIG. 3, the cutting member C is inserted through the cannula 1 of the cannula member A in a detachably attachable manner. The cutting member C is adapted to have a main body 20, which is formed of a round bar-shaped solid material and is satisfactorily longer than the cannula member A, and a cutting blade 21 provided at an end of the main body 20.

The outer diameter of the main body 20 has a size slightly smaller than the inner diameter of the cannula 1 constituting the cannula member A, and is formed into a substantially straight needle shape. Namely, a doctor grips a tail portion 20a on the opposite side of the end provided with the cutting blade 21 and performs pulling-out operation, so that the cutting member C can be easily eliminated from the cannula 1.

However, when a dimensional difference of the outer diameter of the main body 20 with respect to the inner diameter of the cannula 1 is too large, the cannula 1 is easily released, and thus it is not preferable. When a difference between the outer diameter and the inner diameter is too small, the operation at the time of inserting the cutting member C through the cannula 1 or the operation of pulling out the cutting member C from the cannula 1 is not easy, and thus it is not preferable. Accordingly, the outer diameter of the main body 20 can be slightly smaller than the inner diameter of the cannula 1. The tail portion 20a side that does not enter into the cannula 1 may have a straight needle shape continuing from the end side or a different shape such as a tapered shape, and the outer diameter is not limited.

When the cannula 1 has flexibility and deformability, the outer diameter of the main body 20 can be made larger than the inner diameter of the cannula 1.

In the cutting member C, both a solid needle and a hollow needle can be used, and needles having various needlepoint shapes, such as a suture needle and an injection needle, can be used. When the needlepoint shape of the suture needle is used, the cutting blade 21 can have a straight-line shape as viewed from the end side. However, the cutting blade 21 is not necessarily formed to coincide with the straight line passing through the center of the main body 20. The shape of the cutting blade 21 may be a straight line passing through a position displaced from the center or a smooth curved line passing through a position displaced from the center, or may be a combination of straight lines or a combination of a straight line and a smooth curved line.

A material constituting the cutting member C is not particularly limited, and a piano wire, a steel wire, a stainless steel wire rod, and so on can be selectively used. However, when a risk of rust in distribution process is considered, the stainless steel wire rod with a high level of rust resistance is preferably used. Especially, there can be used a wire rod formed of austenitic stainless steel having high reliability with respect to biocompatibility. Furthermore, when an austenitic stainless steel wire rod having a predetermined diameter is subjected to cold drawing process with a reduction in area previously set, the structure of the wire rod is elongated in a fiber shape. The resultant wire rod has suitable elasticity and flexibility and a high toughness, and therefore, it is preferable.

The present inventor attempted a comparative experiment using a trocar having the cannula 1 according to the present invention and the cannula described in the Patent Document 2. In the experiment, the inner diameter and the outer diameter of the cannula are the same in the both cannulas, cutting members having the same specifications are inserted through the cannulas, and the size of projection of the cutting member from the cannula end is the same in the both cannulas. Further, resistance (load N) at the time when test materials having the same coating are impaled was measured.

As a result, in the trocar described in the Patent Document 2 (the trocar of the comparative example), after the start of impalement of the cannula through the test material, the load was increased by 0.797 N while the cannula passes by 0.5 mm. Meanwhile, in the present invention, the load increased during the passage of the cannula by 0.5 mm was 0.363 N. The maximum load at the time of penetration of the test material was 3.5 N in both of the cases.

Accordingly, the rate of the increase in resistance of the cannula of the comparative example is about 2.2 times that of the cannula of the present invention. The position where the resistance with the passage of the cannula through the test material changes to increase is the same in the both cannulas. However, with regard to the position where the maximum load occurs, the position in the cannula of the present invention is more rearward than the position in the cannula of the comparative example, and a difference of about 0.8 mm is found between the two positions.

The above result is due to that while the cannula of the comparative example forms a taper based on the central axis of an oblique cut surface formed at the end of the cannula, the cannula of the present invention forms a taper with uneven width, where the width on the protruding side of the inclined cut surface formed at the end of the cannula is larger than the width on the nonprotruding side.

As described above, in the cannula of the present invention, the rate of the increase in resistance is small when the cannula is operated by a doctor to make it penetrated through an incisional wound in tissue. Accordingly, since an excessive force is not required to be applied, the cannula can be operated in a stable state.

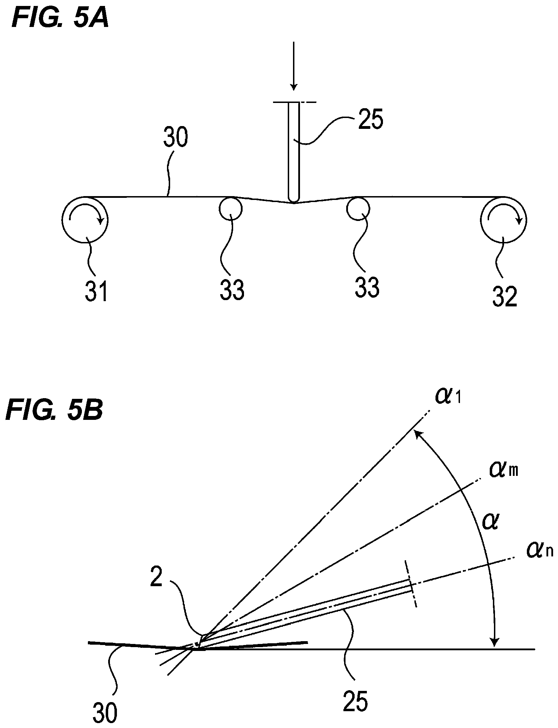

Next, a method of manufacturing the cannula having the above constitution will be described using FIGS. 4 and 5, which is a method of forming a tapered surface, having uneven width where the width of the tapered surface on the protruding side of the inclined cut surface is larger than the width of the tapered surface on the nonprotruding side, on the outer peripheral surface of the inclined cut surface formed at the end of the cylindrical material so as to manufacture the cannula 1 illustrated in FIG. 4.

When the cannula 1 is manufactured, there is provided a material 25 in which a cylindrical material coinciding with the material of the intended cannula 1 and the inner and outer diameters is previously cut into a length corresponding to the length of the intended cannula 1. One end of the material 25 is cut at a previously set angle, and the inclined cut surface 2 is formed.

The outer peripheral surface of the inclined cut surface 2 of the cylindrical material 25 prepared as described above is ground or polished so as to form the tapered portion 3 on the outer peripheral surface. When the tapered portion 3 is formed on the outer peripheral surface of the cylindrical material 25, whether the cylindrical material 25 is ground or polished is not limited. Whether the cylindrical material 25 is ground or polished can be suitably determined depending on the conditions such as the material of the cylindrical material 25 and the outer diameter size. In the present embodiment, the cylindrical material 25 is polished to thereby form the tapered portion 3.

FIG. 5 is a view schematically illustrating a grinding device for manufacturing the cannula 1. As illustrated in FIG. 5, the grinding device is adapted to have a flexible grinding belt 30, a supply portion 31 for extending the grinding belt 30, a winding portion 32 for winding the grinding belt 30, and a transfer portion 33 for transferring the grinding belt 30 at a substantially constant speed.

The grinding belt 30 may be adapted so that the belt can be continuously transferred at a constant speed in a constant direction, and may be adapted so that the belt can move in a certain direction while reciprocally moving at a constant speed within a given length range.

Specifically, in the grinding device, a member for supporting the grinding belt 30 is not provided at a portion where the material 25 is brought into press contact with the grinding belt 30. Accordingly, when the material 25 is brought into press contact with the grinding belt 30, deflection corresponding to a pressure contact force is generated in the grinding belt 30. Then, grinding corresponding to the deflection is performed.

In the grinding belt 30, a grinding material is secured to one side surface of a continuous flexible belt. The grinding belt 30 is wound around the supply portion 31 in an unused state. The material of the flexible belt constituting the grinding belt 30 and the specifications, such as the material and grain size of the grinding material secured to the belt, are not limited. The material of the flexible belt constituting the grinding belt 30 and the specifications can be suitably set depending on the conditions such as the material and outer diameter of the cylindrical material 25.

The supply portion 31 is adapted to have a reel which winds therearound the grinding belt 30 in an unused state and a driving member which drives the reel in the arrow direction. The supply portion 31 is adapted to allow to extend and supply the grinding belt 30 with the progression of the grinding of the cylindrical material 25 by means of the grinding belt 30.

The winding portion 32 is adapted to have a reel which reels the used grinding belt 30 and a driving member which drives the reel. The winding portion 32 is adapted to allow to reel the used grinding belt 30 transferred with the progression of the grinding of the cylindrical material 25 by means of the grinding belt 30.

The transfer portion 33 transfers the grinding belt 30 at a substantially constant speed and has a function of applying a constant torque to the grinding belt 30 and applying a constant tension to the grinding belt 30. Any transfer portion may be used as long as it has such a function. An example of the transfer portion having the function is a mechanism in which a roller pair including a pair of rollers holding the grinding belt 30 therebetween is offset by a previously set distance, and the roller pair can be driven based on a constant rotation number. Another example is a transfer portion configured so that a pair of load cells is arranged and offset by a previously set distance, and the supply portion 31 and/or the winding portion 32 can be driven corresponding to the output of the load cell. Furthermore, the transfer portion may be adapted to use a torque motor as a driving motor which drives the roller pair.

Since the grinding belt 30 is transferred at a substantially constant speed by the transfer portion 33, the cylindrical material 25 can be always ground by a new grinding material. Therefore, the reliable and stable grinding can be realized.

A robot mechanism (not illustrated) is arranged at a position facing the grinding belt 30. The robot mechanism has a chuck which grips the end side of the cylindrical material 25 on the opposite side of the end formed with the inclined cut surface 2. The chuck is adapted to be rotatable and be able to change the posture with respect to the grinding belt 30. Namely, the chuck is adapted to enable to change the inclination angle relative to the grinding belt 30 of the material 25 gripped by the chuck (the angle of the axial center of the cylinder relative to a ground surface or a polished surface) and to enable to change a position in a height direction and a coming and going direction relative to the grinding belt 30.

There will be described a procedure of grinding the cylindrical material 25 in which the inclined cut surface 2 is formed at the end by the grinding device configured as above and forming the tapered portion 3 on the outer peripheral surface of the inclined cut surface 2.

First, the winding portion 32 is driven and the transfer portion 33 is operated to transfer the grinding belt 30. At the same time, the cylindrical material 25 is gripped by a chuck (not illustrated). In this state, as illustrated in FIG. 5A, the material 25 is held vertically to the transfer direction of the grinding belt 30. At the same time, as illustrated in FIG. 5B, the material 25 is held in such a posture that the material 25 is inclined at an angle α₁ (for example, 45 degrees) with respect to the width direction of the grinding belt 30.

Thereafter, the chuck is rotated to rotate the cylindrical material 25 around the axial center, and meanwhile, the cylindrical material 25 is lowered to bring the point 2a of the inclined cut surface 2 into press contact with the grinding belt 30. By virtue of the press contact, deflection illustrated in FIG. 5 is generated in the grinding belt 30. Then, when the end of the material 25 is in press contact with the grinding belt 30, and the grinding belt 30 is rotated by a previously set number of times, the outer peripheral surface near the point 2a including the point 2a is ground into a curved surface shape, so that a portion of the tapered portion 3 can be formed.

When the chuck completes a predetermined number of rotations, the chuck is raised to offset the material 25 from the grinding belt 30. After the chuck is raised to offset the cylindrical material 25 from the grinding belt 30, the inclination angle of the chuck is changed to a second angle (for example, 40 degrees) smaller than the angle α₁, and the position of the chuck in the width direction of the grinding belt 30 is changed, so that the point 2a constituting the inclined cut surface 2 of the material 25 is located closer to the width direction center side of the grinding belt 30.

Thereafter, the chuck is rotated to rotate the cylindrical material 25 around the axial center, and meanwhile, the cylindrical material 25 is lowered, so that the outer peripheral surface of the inclined cut surface 2 already formed with a portion of the tapered portion 3 is in press contact with the grinding belt 30. The deflection is generated in the grinding belt 30 by the press contact. When the end of the material 25 is in press contact with the grinding belt 30 and the grinding belt 30 has been rotated by a previously set number of times, the outer peripheral surface of the inclined cut surface 2 can be ground into a curved surface shape, including a portion of the already formed tapered portion 3. Namely, the tapered portion 3, in which a portion of the tapered portion 3 formed in a posture at the angle α₁ is elongated continuously, can be formed.

When the chuck completes a predetermined number of rotations, the chuck is raised to offset the material 25 from the grinding belt 30. After that, the inclination angle of the chuck is changed to an angle αₘ (for example, 35 degrees) smaller than the second angle, and the position of the chuck in the width direction of the grinding belt 30 is changed, so that the point 2a constituting the inclined cut surface 2 of the material 25 is located closer to the width direction center side of the grinding belt 30. After that, as described above, the chuck is rotated to rotate the cylindrical material 25 around the axial center, and meanwhile, the cylindrical material 25 is lowered. The outer peripheral surface of the inclined cut surface 2 on which a portion of the tapered portion 3 is already formed is brought into press contact with the grinding belt 30, and the grinding belt 30 is rotated by a previously set number of times. Accordingly, the outer peripheral surface of the inclined cut surface 2 can be ground into a curved surface shape, including a portion of the already formed tapered portion 3, and the tapered portion 3, in which a portion of the tapered portion 3 formed in a posture at the second angle is further elongated, can be formed.

While the inclination angle α of the chuck is changed to become smaller in order, from the angle α₁ to the angle αₘ, the position in the width direction of the grinding belt 30 is changed. The chuck is then lowered while being rotated to bring the outer peripheral surface of the inclined cut surface 2 formed at the cylindrical material 25 into press contact with the grinding belt 30 to grind the outer peripheral surface of the inclined cut surface 2. Repeating the series of operation enables the tapered portion 3 formed on the outer peripheral surface of the inclined cut surface 2 to be elongated. When the inclination angle α of the chuck is changed to become smaller in order, from the angle α₁ to the angle αₘ, the angle may be changed in a stepwise manner or in a continuous manner.

Specifically, as the inclination angle of the chuck becomes smaller, the point 2a constituting the inclined cut surface 2 and the vicinity including the points 2a are offset from the grinding belt 30, and grinding is not progressed. When the inclination of the chuck is set to the smallest angle αₙ (for example, 25 degrees) to bring the cylindrical material 25 into press contact with the grinding belt 30, the ground portion of the material 25 ground by the grinding belt 30 is located offset from the inclined cut surface 2. By virtue of the grinding at that time, the tapered portion 3 and the outer peripheral surface of the cylinder portion of the material 25 are smoothly continued through the cut-out line 4.

Accordingly, although the cut-out line 4 is described as a clear line in FIGS. 1 to 4, in the actual cannula 1, the cut-out line 4 does not appear as a clear discontinuous portion.

As described above, by virtue of the use of the flexible grinding belt 30, when the end of the cylindrical material 25 to be ground is in press contact with the grinding belt 30, deflection is generated in the grinding belt 30. The material 25 can be ground so that the press contact portion of the material 25 is encompassed by the deflected grinding belt 30. Namely, the end of the material 25 to be ground is not ground linearly but can be ground in a curved surface manner.

Accordingly, the outer peripheral surface of the inclined cut surface 2 formed at the material 25 is ground so that the inclination angle α of the material 25 relative to the grinding belt 30 is changed to become smaller in sequence, so that the outer peripheral surface of the inclined cut surface 2 is formed into a curved surface shape having a large curvature at the point 2a and in the vicinity including the point 2a, into a curved surface shape having a smaller curvature at the point 2b and in the vicinity including the point 2b, and into a curved surface shape having an even smaller curvature at a portion offset in the longitudinal direction of the material 25 relative to the point 2b.

As described above, because the grinding belt 30 has flexibility, the curved surfaces having different curvatures smoothly continue to each other to constitute the tapered portion 3. Especially, the inclination angle of the material 25 at the time of grinding at the final stage is made extremely small, so that the cut-out line 4, that is a border between the tapered portion 3 and the outer peripheral surface of the cylinder portion of the material 25, can be formed at a substantially right angle to the axial center of the material 25 (the axial center 1a of the cannula 1).

Accordingly, the tapered portion 3 with an uneven tapered width, where the dimension from the point 2a to the cut-out line 4 (width on the protruding side) is larger than the dimension from the point 2b to the cut-out line 4 (width on the nonprotruding side), can be provided.

By going through the above processes, the cannula 1 can be manufactured from the cylindrical material 25.

### INDUSTRIAL APPLICABILITY

In the present embodiment, although the cannula used in an eye surgery and the method of manufacturing the cannula have been described, the cannula can be utilized in endoscopic surgery as the cannula penetrated through an incisional wound formed in surface tissue, and the method can be utilized when such a cannula is manufactured.

## Claims

1. A cylindrical cannula (1) which is a part of a trocar (B) used in a surgery and into which a cutting member (C) is inserted in detachably attachable manner, comprising:
an inclined cut end provided at an end of the cylindrical cannula (1); and
a circumferential surface (3) provided at the periphery of the inclined cut end, the circumferential surface (3) being tapered,
wherein the inclined cut end has a tip such that the circumferential surface (3) has a longest length along the longitudinal direction of the circumferential surface (3) of the cannula (1) at the tip,
being **characterized in that** the circumferential surface (3) has uneven lengths along the axial direction of the cannula (1), and
**in that** the circumferential surface (3) has a decrease in rate of an increase in diameter in a direction from the inclined cut end to the other end of the cylindrical cannula (1) .

## Patentansprüche

1. Zylindrische Kanüle (1), welche ein Teil eines Trokars (B) ist, der in der Chirurgie verwendet wird, und in welche ein Schneideteil (C) lösbar befestigbar eingesetzt wird, umfassend:
ein geneigtes Schnittende, das an einem Ende der zylindrischen Kanüle (1) vorgesehen ist; und
eine Umfangsfläche (3), die am Rand des geneigten Schnittendes vorgesehen ist, wobei die Umfangsfläche (3) spitz zuläuft,
wobei das geneigte Schnittende eine Spitze aufweist, sodass die Umfangsfläche (3) eine längste Länge entlang der Längsrichtung der Umfangsfläche (3) der Kanüle (1) an der Spitze aufweist,
**dadurch gekennzeichnet, dass** die Umfangsfläche (3) ungleiche Länge entlang der Axialrichtung der Kanüle (1) aufweist, und
dadurch, dass die Umfangsfläche (3) eine Verringerung in Rate der Durchmesserzunahme in eine Richtung von dem geneigten Schnittende zu dem anderen Ende der zylindrischen Kanüle (1) aufweist.

## Revendications

1. Canule cylindrique (1), laquelle fait partie d'un trocart (B) utilisé en chirurgie et dans laquelle un membre coupant (C) est inséré de manière à être fixé de manière amovible, comprenant :
une extrémité coupée inclinée prévue à une extrémité de la canule cylindrique (1) ; et
une surface circonférentielle (3) prévue à la périphérie de l'extrémité coupée inclinée, la surface circonférentielle (3) étant effilée,
dans laquelle l'extrémité coupée inclinée possède une pointe telle que la surface circonférentielle (3) possède une longueur la plus longue le long de la direction longitudinale de la surface circonférentielle (3) de la canule (1) à la pointe,
étant **caractérisée en ce que** la surface circonférentielle (3) possède des longueurs inégales le long de la direction axiale de la canule (1), et
**en ce que** la surface circonférentielle (3) a une diminution du taux d'augmentation de diamètre dans une direction allant de l'extrémité coupée inclinée à l'autre extrémité de la canule cylindrique (1).
